(19) Europäisches Patentamt
European Patent Office
Office européen des brevets

(11) Veröffentlichungsnummer: **0 178 260**
**B1**

(12) # EUROPÄISCHE PATENTSCHRIFT

(45) Veröffentlichungstag der Patentschrift: **16.05.90**

(51) Int. Cl.$^5$: **C 07 D 213/61,** C 07 D 213/73
// C07D213/64

(21) Anmeldenummer: **85810455.7**

(22) Anmeldetag: **04.10.85**

(54) **Verfahren zur Herstellung von fluorierten Pyridin-Derivaten.**

(30) Priorität: **10.10.84 CH 4855/84**

(43) Veröffentlichungstag der Anmeldung:
**16.04.86 Patentblatt 86/16**

(45) Bekanntmachung des Hinweises auf die Patenterteilung:
**16.05.90 Patentblatt 90/20**

(84) Benannte Vertragsstaaten:
**AT BE CH DE FR GB IT LI NL**

(56) Entgegenhaltungen:
**EP-A- 97 460**
**DE-A-2 409 379**
**DE-A-3 234 451**
**FR-A-2 359 106**
**GB-A-1 344 636**
**GB-A-2 039 473**
**GB-A-2 053 189**
**US-A-3 703 521**
**US-A-4 031 100**
**US-A-4 071 521**
**Berrie et al, J. Chem. Soc. 1952 2042**
**J. Fluor. Chem. 1982, 171-89**
**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Klybeckstrasse 141**
**CH-4002 Basel (CH)**

(72) Erfinder: **Schurter, Rolf, Dr.**
**Holzmattstrasse 45**
**CH-4102 Binningen (CH)**
Erfinder: **Siegrist, Urs**
**Rüttenenweg 61**
**CH-4313 Möhlin (CH)**
Erfinder: **Rempfler, Hermann, Dr.**
**Brücklismattstrasse 16**
**CH-4107 Ettingen (CH)**
Erfinder: **Baumeister, Peter**
**St. Annaweg 24**
**CH-4113 Flüh (CH)**

**EP 0 178 260 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein neues Verfahren zur Herstellung von 2,3-Difluoropyridinen.

2,3-Difluorpyridine sind wertvolle Zwischenprodukte für die Herstellung von Herbiziden aus der Klasse der Pyridinyloxyphenoxyalkancarbonsäure-Derivate. Derartige herbizide Wirkstoff sind mit ihren biologischen Eigenschaften in den publizierten Europäischen Patentanmeldungen EP—A—83556 und EP—A—97460 beschrieben.

Die in den publizierten Europäischen Patentanmeldungen EP—A—97460 und EP—A—104715 beschriebenen Verfahren zur Herstellung von 2,3-Difluorpyridinen erweisen sich für den grosstechnischen Einsatz als wenig geeignet, weil einerseits teuere Chemikalien wie Cäsiumfluorid mindestens in stöchiometrischen Mengen benötigt werden, aber andererseits gewünschte Produkte nur in unbefriedigenden Ausbeuten erhalten werden.

Aus den Patentpublikationen DE—A—2 409 379, US 3,703,521 und FR—A—2 359 106 sind Verfahren zur Herstellung von Fluorpyridinen nach dem Schiemann Verfahren bekannt geworden, bei denen in reinem (wasserfreiem) Fluorwasserstoff als Lösungsmittel gearbeitet wird. Die Schiemann Reaktion wird dabei in zwei Verfahrensschritten durchgeführt. In der ersten Verfahrensstufe wird ein Aminopyridin bei Temperaturen von −20 bis + 25°C (DE—A—2 409 379), bei −10°C (US 3.703.521) oder bei −20 bis +10°C (FR—A—2 359 106) diazotiert und dann das so erhältiche Diazoniumsalz in einer zweiten Verfahrensstufe bei 0 bis 100°C (DE—A—2 409 379, US 3,703,521) oder bei 40 bis 50°C (FR—A—2 359 106) in einem Autoklaven zersetzt.

Aus den Dokumenten GB 2 039 473, US 4,071,521 und US 4,031,100 sind Verfahren zum Halogen-Fluor-Autausch an Pyridinen bekannt geworden. Als Verfahrensmassnahmen zur Verbesserung der Gesamtausbeute werden unter anderem katalytische Beimengungen von Eisen-, Nickel- oder Kupfer-Halogeniden (GB 2 039 473), die Umsetzung von Chlor- oder Dichlorpyridinen in DMSO unter Verwendung von im wesentlichen fluorwasserstofffreien Edukten mir Fluorid in Gegenwart von Kaliumcarbonat oder Kaliumhydrogencarbonat (US 4,071,521) oder aber die Umsetzung von 2,6-Dichlorpyridin mit Kaliumfluorid in DMSO unter Phasentransferkatalyse quaternärer Ammonium- oder Phosphoniumsalze (US 4,031,100), vorgeschlagen.

Es besteht ein Bedürfnis nach einem einfachen Verfahren zur Herstellung der 2,3-Difluorpyridin-Zwischenprodukte, das es ermöglicht, diese Produkte unter Einsatz billigerer Reagenzien in höheren Ausbeuten herzustellen.

Ueberraschenderweise wurde nun ein neues Verfahren zur Herstellung dieser wertvollen Zwischenprodukte gefunden, das dieses Bedürfnis weitgehend befriedigt.

Gemäss vorliegender Erfindung wird daher vorgeschlagen, 2,3-Difluorpyridine der Formel I

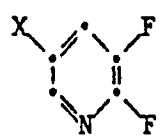

(I),

worin X für Halogen oder Trifluormethyl steht, herzustellen, indem man ein 3-Amino-2-halogenpyridin der Formel II

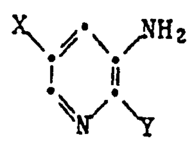

(II),

worin X die unter Formel I gegebene Bedeutung hat und Y für Brom, Chlor oder Fluor steht, durch Diazotieren in Gegenwart von Fluorwasserstoff in einem inertem Lösungsmittel in ein 3-Fluor-2-halogenpyridin der Formel III

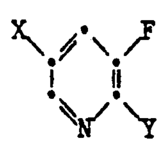

(III),

worin X und Y die unter Formel II gegebene Bedeutung haben, überführt, und erhaltene 3-Fluor-2-halogenpyridine der Formel III, in welchen Y Brom oder Chlor bedeutet, mit einem Fluorierungsmittel in Gegenwart einer katalytischen Mange eines Gemischs aus Cäsiumfluorid und einem Kronenäther behandelt.

In den Formeldefinitionen steht Halogen für Fluor, Chlor, Brom oder Jod, vorzugsweise für Fluor, Chlor oder Brom, jedoch insbesondere für Chlor.

Vorzugsweise wird das erfindungsgemässe Verfahren eingesetzt, um die Verbindungen der Formel I herzustellen, worin X für Chlor oder Trifluormethyl steht. Entsprechend werden bevorzugt solche Ausgangsmaterialien der Formel II verwendet, bzw. Zwischenprodukte der Formel III durchlaufen, in denen X für Chlor oder Trifluormethyl steht. Für die Durchführung des Verfahrens ist es von Vorteil, wenn der Substituent Y in den Ausgangs- und Zwischenprodukten der Formeln II und III für Chlor oder Fluor steht. Insbesondere eignen sich deshalb für die Durchführung des erfindungsgemässen Verfahrens die Verbindungen der Formel II, worin X Chlor oder Trifluormethyl und Y Chlor oder Fluor bedeuten. Entsprechend wird das Zwischenprodukt der Formel III durchlaufen, worin X Chlor oder Trifluormethyl und Y Chlor bedeuten.

Bei Verwendung von 3-Amino-2-halogenpyridinen der Formel II, in welchen Y Fluor bedeutet, werden die 2,3-Difluorpyridine der Formel I durch Diazotieren in Gegenwart von Fluorwasserstoff direkt erhalten. Bei Verwendung von 3-Amino-2-halogenpyridinen der Formel II, in welchen Y Chlor oder Brom bedeutet, wird das erfindungsgemässe Verfahren im allgemeinen in zwei aufeinanderfolgenden Reaktionsstufen in der Weise durchgeführt, dass in der ersten Stufe die Aminofunktion in der 3-Stellung mit einem Nitrit diazotiert und gegen Fluor ausgetauscht wird, während in der zweiten Stufe das Chlor- oder Bromatom in der 2-Stellung mittels eines Fluorierungsmittels durch Fluor ersetzt wird.

Die Diazotierung auf der ersten Reaktionsstufe führt man mit Vorteil in Gegenwart eines Ueberschusses von Fluorwasserstoff aus. Mindestens erforderlich ist ein Aequivalent Fluorwasserstoff. Dabei wird ein inertes Lösungsmittel verwendet, wie z.B. Sulfolan, Dimethylsulfoxid, Amide, wie Dimethylformamid, Dimethylacetamid, Dimethylpropylenharnstoff, oder Aether wie Tetraäthylenglykoldimethyläther. Dabei wird die Reaktionstemperatur so gewählt, dass sich das Diazoniumfluorid bildet und gleichzeitig Stickstoff abspaltet. Der Einsatz dieser inerten Lösungsmittel ermöglicht eine Senkung des Dampfdrucks des Fluorwasserstoffs. Durch die damit verbundene Druckminderung kann dann bei Normaldruck gearbeitet werden. Damit werden die betriebswirtschaftlichen Kosten für den Reaktor gesenkt. Bei dieser Verfahrensweise wird die Reaktionstemperatur zwischen 0°C und +70°C gewählt.

Als Diazotierungsmittel werden im allgemeinen Nitrite verwendet; allgemein üblich sind Natrium- oder Kaliumnitrit, aber auch Distickstofftrioxid, salpetrige Säure, Nitrosylhalogenide oder Komplexe von Nitrosylhalogeniden mit dem Reaktionspartner Fluorwasserstoff. Bevorzugt sind Natriumnitrit oder Distickstofftrioxid.

Die Substitution eines Chlor- oder Bromatoms in der 2-Stellung des Pyridinkörpers durch Fluor wird im allgemeinen in einem inerten polaren aprotischen Lösungsmittel, wie Dimethylsulfoxid, Dimethylsulfon, N-Methylpyrrolidinon, Dimethylacetamid, Dimethylformamid, Sulfolan oder Hexamethylphosphorsäuretriamid, durchgeführt. Die Reaktionstemperaturen liegen allgemein zwischen 80°C und 220°C. Bovorzugt sind Temperaturen zwischen 120°C und 170°C. Als Fluorierungsmittel hat sich Kaliumfluorid als besonders geeignet erwiesen. Man setzt es in mindestens äquimolarer Menge ein. Eine weitere sich zum Vorteil der Reaktionsführung auswirkende Massnahme ist der Einsatz eines Fluorierungskatalysators. Solche Katalysatoren sind einerseits schwere Alkalimetallfluoride wie Cäsiumfluorid gemeinsam mit einem Kronenäther wie 12-Crown-4, 15-Crown-5, 18-Crown-6, Dibenzo-18-crown-6, Dicyclohexano-18-crown-6 oder Dicyclohexano-24-crown-8. Zweckmässig ist ein Katalysatorzusatz von 0,001 bis 0,1 Mol pro Mol zu fluorierendes Ausgangsmaterial.

Die Diazotierung in Gegenwart von Fluorwasserstoff und die Substitution von Chlor oder Brom in 2-Stellung des Pyridinringes durch Fluor können wahlweise nacheinander im gleichen Reaktor ohne Isolierung des Zwischenproduktes oder einzeln unter zwischenzeitlicher Isolierung des Zwischenproduktes der Formel III in verschiedenen Reaktoren durchgeführt werden.

Die Ausgangsmaterialen und Zwischenprodukte der Formeln II und III sind bekannt für den Fall, dass X Trifluormethyl bedeutet.

Die Verbindungen der Formeln II und II, worin X für Halogen steht sind teilweise neu und wurden speziell im Zusammenhang mit dem erfindungsgemässen Verfahren entwickelt und hergestellt. Diese neuen Verbindungen, das heisst Verbindungen der Formel IIa

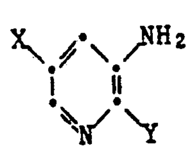

(IIa),

worin X für Halogen und Y für Brom, Chlor oder Fluor stehen, mit Ausnahme von 3-Amino-2,5-dichlorpyridin, 3-Amino-2,5-dibrompyridin, 3-Amino-2-chlor-5-brompyridin und 3-Amino-2-brom-5-chlorpyridin, und Verbindungen der Formel IIIa

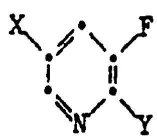

(IIIa),

worin X für Halogen und Y für Brom oder Chlor stehen, mit Ausnahme von 2,3,5-Trifluorpyridin, 2,3-Difluor-5-chlorpyridin und 2,3-Difluor-5-brompyridin, bilden daher einen Bestandteil der vorliegenden Erfindung.

Die Verbindungen der Formel II werden aus den entsprechenden 3-Nitropyridin-Derivaten der Formel IV

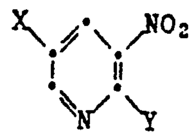

(IV),

worin X und Y die unter Formel II gegebene Bedeutung haben, durch katalytische Reduktion mit Wasserstoff hergestellt. Uebliche dem Fachmann geläufige angewendete Reaktionsbedingungen dafür sind;

Wasserstoffdrucke zwischen 1 und 20 bar, vorzugsweise 1 bis 5 bar;

Reaktionstemperaturen zwischen −20°C und +50°C, vorzugsweise zwischen 0°C und 30°C;

inertes Lösungsmittel aus der Reihe: Aether, Ester, Alkohole oder Kohlenwasserstoff; vorzugsweise; Tetrahydrofuran, Dioxan, Diäthyläther, Aethylacetat, Methanol, Aethanol, Pentan, Cyclohexan oder Benzol; und

Hydrierungskatalysatoren aus der 8. Nebengruppe des Periodischen Systems der Elemente: Nickel, Palladium oder Platin in handelsüblicher Form wie zum Beispiel als Raney-Nickel, Palladium auf Kohle, Platinoxid oder Platinmoor.

Die eingesetzten 3-Nitropyridine der Formel IV sind bekannt oder können nach an sich bekannten Verfahren hergestellt werden.

Aus den erfindungsgemäss hergestellten 2,3-Difluorpyridinen erhält man die wertvollen Herbizide aus der Klasse der 2-[4-(3-Fluorpyridin-2-yloxy)-phenoxy]-propionsäure-Abkömmlinge beispielsweise nach den Reaktionen, wie sie im nachfolgenden Schema 1 zusammengefasst sind:

Schema 1:

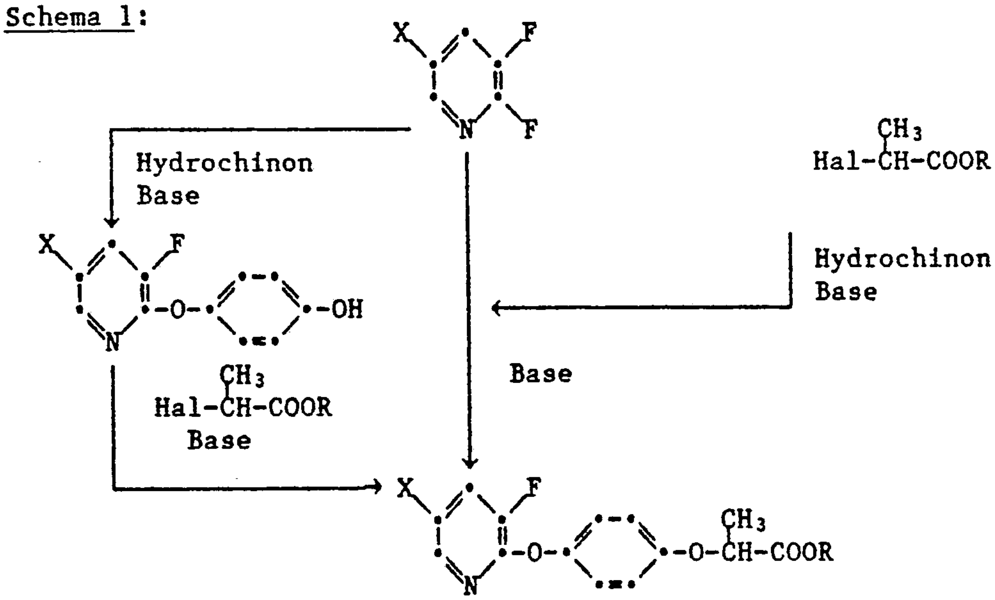


(I)

Hal steht für Halogen, vorzugzweise Chlor oder Brom, R für einen beliebigen organischen Rest.

Die anschliessenden Beispiele dienen der näheren Illustration der vorliegenden Erfindung. Die Beispiele H4 bis H8 können wahlweise die Reaktion der Stufe b des Beispiels H1, das Beispiel H9 die der Stufen b und c des Beispiels H1 ersetzen.

Herstellungsbeispiele:

Beispiel H1

5-Chlor-2,3-difluoropyridin

a) 3-Amino-2,5-dichlorpyridin

26,0 g Raney-Nickel-Katalysator werden mit Aethanol gewaschen und einer Lösung von 129,2 g (0,69 mol) 2,5-Dichlor-3-nitropyridin in 1300 ml Dioxan zugesetzt. Diese Mischung wird unter Normaldruck bei einer Temperatur zwischen 20° und 35°C mit Wasserstoff hydriert. Nach Umsetzung von 20% der erforderlichen Wasserstoffmenge setzt man der Reaktionsmischung weitere 30,0 g Raney-Nickel-Katalysator zu. Nach einer Hydrierdauer von 22 Stunden wird der Katalysator abgetrennt, das Lösungsmittel abgedampft und der Rückstand aus Aethylacetat/Hexan kristallisiert. Man erhält so 84,9 g (78% d.Th.) 3-Amino-2,5-dichlorpyridin, Smp. 129—132°C.

b) 2,5-Dichlor-3-fluorpyridin

In einem Monel-Autoklaven werden 450 ml (22,5 mol) Fluorwasserstoff vorgelegt und bei einer Temperatur zwischen −5°C und −1°C mit 163 g (1,0 mol) 3-Amino-2,5-dichlorpyridin versetzt. Innerhalb von 1,5 Stunden werden in diese Lösung 82,8 g (1,2 mol) Natriumnitrit eingetragen. Nachdem die Reaktionsmischung bei der gleichen Temperatur für 1,5 Stunden gerührt worden ist, wird die Temperatur stufenweise auf +60°C erhöht. Wenn keine Gasentwicklung mehr stattfindet, wird der Fluorwasserstoff abgedampft, der Rückstand in Methylenchlorid aufgenommen und in Eiswasser eingegossen. Das zweiphasige Gemisch wird mit konzentrierter Ammoniaklösung neutralisiert. Die organische Phase wird abgetrennt, die wässrige dreimal mit Methylenchlorid extrahiert. Die vereinigten organischen Phasen werden mit Wasser gewaschen, über Magnesiumsulfat getrocknet, mit Aktivkohle behandelt, durch Kieselgel filtriert und eingedampft. Man erhält so 141,5 g (85% d.Th.) 2,5-Dichlor-3-fluorpyridin, Smp. 38—39°C.

c) 64,6 g (1,11 mol) Kaliumfluorid und 11,25 g (0,074 mol) Cäsiumfluorid werden in 240 ml Sulfolan suspendiert und auf 140°C erhitzt. Durch Druckverminderung destilliert man 50 ml Sulfolan ab und setzt anschliessend der Suspension 61,4 g (0,37 mol) 2,5-Dichlor-3-fluorpyridin und 1,45 g (0,0055 mol) 18-Crown-6 in 20 ml Sulfolan zu. Dieses Reaktionsgemisch wird für 35 Stunden bei einer Temperatur von 140°C gerührt und dann in Eiswasser aufgenommen. Aus dem wasserhaltigen Gemisch wird das Produkt entweder durch Extraktion mit Aether oder durch Wasserdampfdestillation isoliert. Man erhält so 48,7 g (88% d.Th.) 5-Chlor-2,3-difluorpyridin, Sdp. 65—66°C bei 133 mbar.

### Beispiel H2

### 2-[4-(5-Chlor-3-fluorpyridin-2-yl-oxy)-phenoxy]-propionsäuremethylester

Zu einer Mischung von 21,6 g (0,11 mol) 2-(4-Hydroxyphenoxy)-propionsäuremethylester, 15,2 g (0,11 mol) Kaliumcarbonat, 1,45 g (0,0055 mol) 18-Crown-6 und 100 ml Acetonitril lässt man eine Lösung von 14,95 g (0,10 mol) 5-Chlor-2,3-difluorpyridin in 30 ml Acetonitril zutropfen und erhitzt das Reaktionsgemisch für 40 Stunden auf eine Temperatur zwischen 50°C und 60°C. Durch Aufnehmen der Mischung in Eiswasser, Abtrennen der organischen Phase, dreimalige Extraktion der wässrigen Phase mit Aethylacetat, Trocknen und Eindampfen der vereingten organischen Phasen erhält man einen öligen Rückstand, der durch Lösen in Hexan/Aethylacetat-Gemisch und Filtration über Kieselgel gereinigt wird. Durch Verdampfen des Lösungsmittels erhält man aus dem Filtrat 20,4 g (63% d.Th.) 2-[4-(5-Chlor-3-fluorpyridin-2-yloxy)-phenoxy]-propionsäuremethyl-ester, Smp. 58—59°C.

### Beispiel H3

### 2,3-Difluor-5-trifluormethylpyridin

a) 3-Amino-2-chlor-5-trifluormethylpyridin

12,3 g (0,0543 mol) 2-Chlor-3-nitro-5-trifluormethylpyridin werden in 250 ml Aethanol gelöst, 2,4 g Raney-Nickel-Katalysator zugesetzt und unter Normaldruck bei einer Temperatur zwischen 20°C und 25°C mit Wasserstoff hydriert. Nach einer Hydrierdauer von 28 Stunden wird der Katalysator abgetrennt, das Lösungsmittel abdestilliert und der Rückstand aus Aethylacetat/Hexan kristallisiert. Man erhält so 9,0 g (84% d.Th.) 3-Amino-2-chlor-5-trifluormethylpyridin, Smp. 94—95°C.

b) 2-Chlor-3-fluor-5-trifluormethylpyridin

In einem Monel-Autoklaven werden 120 g (6,0 mol) Fluorwasserstoff vorgelegt und bei einer Temperatur zwischen −5°C und 0°C mit 27,0 g (0,137 mol) 3-Amino-2-chlor-5-trifluormethylpyridin versetzt. Innerhalb von einer Stunde werden in diese Lösung 10,35 g (0,15 mol) Natriumnitrit eingetragen. Nachdem die Reaktionsmischung bei der gleichen Temperatur während 2 Stunden gerührt worden ist, wird die Temperatur stufenweise auf 50°C erhöht. Wenn keine Stickstoffentwicklung mehr stattfindet, wird der Fluorwasserstoff abgedampft, der Rückstand in Methylenchorid aufgenommen und in Eiswasser eingegossen. Das zweiphasige Gemisch wird unter guter Kühlung mit konzentrierter Ammoniaklösung neutralisiert. Die organische Phase wird abgetrennt und die wässerige dreimal mit Methylenchlorid extrahiert. Die vereinigten organischen Phasen werden mit Wasser gewaschen, über Magnesiumsulfat getrocknet und durch etwas Kieselgel filtriert. Nach dem Abdestillieren des Lösungsmittels wird das erhaltene Produkt destilliert, Sdp. 113°C (975 mbar).

c) 47,7 g (0,82 mol) Kaliumfluorid und 10,0 g (0,065 mol) Cäsiumfluorid werden in 300 ml Sulfolan suspendiert und auf 140°C erhitzt. Durch Druckverminderung destilliert man 60 ml Sulfolan ab und setzt anschliessend der Suspension 65,6 g (0,329 mol) 2-Chlor-3-fluor-5-trifluormethylpyridin und 1,3 g (0,004 mol) 18-Crown-6 zu. Das Reaktionsgemisch wird für 48 Stunden bei einer Temperatur von 140°C gerührt und anschliessend unter Einleitung von Wasserdamp destilliert. Das Oel wird abgetrennt und die wässerige Phase zweimal mit wenig Aether extrahiert. Die organischen Phasen werden bereinigt, mit

etwas Magnesiumsulfat getrocknet und filtriert. Durch Destillation erhält man 54,8 g (91% d.Th.) 2,3-Difluor-5-trifluormethylpyridin, Sdp. 100—102°C (980 mbar).

Beispiel H4

2,5-Dichlor-3-fluorpyridin

In einem 500 ml Reaktor aus Polytetrafluoräthylen, der mit Rührwerk, Thermometer und Rückflusskühler ausgerüstet ist, werden 120 g Fluorwasserstoff vorgelegt. Bei einer Temperatur zwischen 0°C und +10°C werden 40,7 g (0,25 mol) 3-Amino-2,5-dichlorpyridin gelöst in 60 ml Dimethylsulfoxid zugetropft. Bei einer Temperatur von +40°C werden in diese Lösung 20,7 g (0,3 mol) Natriumnitrit eingetragen. Der entstehende Stickstoff wird über den Rückflusskühler aus dem Reaktor entfernt. Nach 4 Stunden bei +40°C ist die Gasentwicklung beendet. Das Reaktionsgemisch wird mit 150 ml Methylenchlorid versetzt und in Eiswasser eingegossen. Das zweiphasige Gemisch wird mit konzentrierter Ammoniaklösung neutralisiert. Die organische Phase wird abgetrennt, die wässrige Phase dreimal mit Methylenchlorid extrahiert. Die vereinigten organischen Phasen werden mit Wasser gewaschen, über Natriumsulfat getrocknet und durch Kieselgel filtriert. Durch destillative Trennung der Lösung erhält man 36,5 g (88% d.Th.) 2,5-Dichlor-3-fluorpyridin.

Beispiel H5

2,5-Dichlor-3-fluorpyridin

In einem 500 ml Reaktor aus Polytetrafluoräthylen, der mit Rührwerk, Thermometer und Rückflussigkühler ausgerüstet ist, werden 60 g Fluorwasserstoff vorgelegt. Bei einer Temperatur zwischen 0°C und +10°C werden 40,7 g (0,25 mol) 3-Amino-2,5-dichlorpyridin gelöst in 100 ml Sulfolan zugetropft. Bei einer Temperatur von +50°C werden in diese Lösung 20,7 g (0,3 mol) Natriumnitrit eingetragen. Der entstehende Stickstoff wird über den Rückflusskühler aus dem Reaktor entfernt. Nach 2 Stunden bei +50°C ist die Gasentwicklung beendet. Das Reaktionsgemisch wird mit 150 ml Methylenchlorid versetzt und in Eiswasser eingegossen. Das zweiphasige Gemisch wird mit konzentrierter Ammoniaklösung neutralisiert. Die organische Phase wird abgetrennt, die wässrige Phase mehrmals mit Methylenchlorid extrahiert. Die vereinigten organischen Phasen werden über Natriumsulfat gertrocknet und eingedampft. Durch destillative Trennung der Sulfolan-Produkt-Lösung erhält man 34,3 g (82,6% d.Th.) 2,5-Dichlor-3-fluorpyridin.

Beispiel H6

2,5-Dichlor-3-fluorpyridin

In einem 500 ml Reaktor aus Polytetrafluoräthylen, der mit Rührwerk, Thermometer und Rückflusskühler ausgerüstet ist, werden 120 g Fluorwasserstoff vorgelegt. Bei einer Temperatur zwischen 0°C und +10°C werden 40,7 g (0,25 mol) 3-Amino-2,5-dichlorpyridin gelöst in 100 ml Dimethylformamid zugetropft. Bei einer Temperatur von 55°C werden in diese Lösung 20,7 g (0,3 mol) Natriumnitrit eingetragen. Der entstehende Stickstoff wird über den Rückflusskühler aus dem Reaktor entfernt. Nach 2 Stunden bei +50°C ist die Gasentwicklung beendet. Das Reaktionsgemisch wird mit 150 ml Methylenchlorid versetzt und in Eiswasser eingegossen. Das zweiphasige Gemisch wird mit konzentrierter Ammoniaklösung neutralisiert. Die organische Phase wird abgetrennt, die wässrige Phase mehrmals mit Methylenchlorid extrahiert. Die vereinigten organischen Phasen werden über Natriumsulfat getrocknet und eingedampft. Nach destillativer Reinigung des Rohprodukts erhält man 37,9 g (91,4% d.Th.) 2,5-Dichlor-3-fluorpyridin.

Beispiel H7

2,5-Dichlor-3-fluorpyridin

In einem 500 ml Reaktor aus Polytetrafluoräthylen, der mit Rührwerk, Thermometer und Rückflusskühler ausgerüstet ist, werden 120 g Fluorwasserstoff vorgelegt. Bei einer Temperatur zwischen 0°C und +10°C werden 40,7 g (0,25 mol) 3-Amino-2,5-dichlorpyridin gelöst in 100 ml Dimethylacetamid zugetropft. Bei einer Temperatur von 55°C werden in diese Lösung 20,7 g (0,3 mol) Natriumnitrit eingetragen. Der entstehende Stickstoff wird über den Rückflusskühler aus dem Reaktor entfernt. Nach 2,5 Stunden bei 55°C ist die Gasentwicklung beendet. Das Reaktionsgemisch wird mit 150 ml Methylenchlorid versetzt und in Eiswasser gegossen. Das zweiphasige Gemisch wird mit konzentrierter Ammoniaklösung neutralisiert. Die organische Phase wird abgetrennt, die wässrige Phase mehrmals mit Methylenchlorid extrahiert. Die vereinigten organischen Phasen werden über Natriumsulfat getrocknet und eingedampft. Nach destillativer Reinigung des Rohprodukts erhält man 36,4 g (87,8% d.Th.) 2,5-Dichlor-3-fluorpyridin.

Beispiel H8

2,5-Dichlor-3-fluorpyridin

In einem 500 ml Reaktor aus Polytetrafluoräthylen, der mit Rührwerk, Thermometer und Rückflusskühler ausgerüstet ist, werden 120 g Fluorwasserstoff vorgelegt. Bei einer Temperatur zwischen 0°C und +10°C werden 40,7 g (0,25 mol) 3-Amino-2,5-dichlorpyridin gelöst in 60 ml Dimethylsulfoxid zugetropft. Bei einer Temperatur von +50°C bis 60°C werden in diese Lösung 24,7 g (0,325 mol) Distickstofftrioxid innert 1.5 Stunden eingeleitet. Der entstehende Stickstoff wird über den Rückflusskühler aus dem Reaktor entfernt. Nach insgesamt 3 Stunden bei 50°C bis 60°C ist die Gasentwicklung beendet. Das

Reaktionsgemisch wird mit 150 ml Methylenchlorid versetzt und in Eiswasser gegossen. Das zweiphasige Gemisch wird mit konzentrierter Ammoniaklösung neutralisiert. Die organische Phase wird abgetrennt, die wässrige Phase mehrmals mit Methylenchlorid extrahiert. Die vereinigten organischen Phasen werden über Natriumsulfat getrocknet. Nach destillativer Trennung der Lösung erhält man 38,2 g (92,1% d.Th.) 2,5-Dichlor-3-fluorpyridin.

Beispiel H9
5-Chlor-2,3-difluorpyridin

In einem 500 ml Reaktor aus Polytetrafluoräthylen, der mit Rührwerk, Thermometer und Rückflusskühler ausgerüstet ist, werden 120 g Fluorwasserstoff vorgelegt. Bei einer Temperatur zwischen 0°C und +10°C werden 40,7 g (0,25 mol) 3-Amino-2,5-dichlorpyridin gelöst in 100 ml Sulfolan zugetropft. Bei einer Temperatur von +50°C werden in diese Lösung 20,7 g (0,3 mol) Natriumnitrit eingetragen. Der entstehende Stickstoff wird über den Rückflusskühler aus dem Reaktor entfernt. Nach 2 Stunden bei +50°C ist die Gasentwicklung beendet. Das Reaktionsgemisch wird mit 150 ml Methylenchlorid versetzt und auf Eiswasser gegossen. Das zweiphasige Gemisch wird mit Ammoniaklösung neutralisiert. Die organische Phase wird abgetrennt und über Natriumsulfat getrocknet. Das Lösungsmittel wird abdestilliert und das Sulfolan/Produktgemisch wird mit 200 ml Sulfolan, 166 g (2,879 mol) Kaliumfluorid und 2 g Tetrabutylammoniumbromid versetzt. Dieses Reaktionsgemisch wird für 7 Stunden bei 179°C gerührt. Anschliessend wird das Produkt direkt aus der Sulfolan-Lösung abdestilliert. Nach destillativer Reinigung des Rohprodukts erhält man das 5-Chlor-2,3-fluorpyridin, Sdp. 137—139°C.

Beispiel H10
3-Amino-5-chlor-2-fluor-pyridin

7.5 g (0.042 mol) 5-Chlor-2-fluor-3-nitro-pyridin werden in 80 ml Ethanol gelöst und in Gegenwart von 1 g Raney-Nickel-Katalysator unter Normaldruck bei einer Temperatur zwischen 20° und 25°C mit Wasserstoff hydriert. Nach einer Hydrierdauer von 20 Stunden wird der Katalysator abgetrennt und das Lösungsmittel abgedampft. Der Rückstand wird in Ethylacetat aufgenommen, durch eine Kieselgel-Schicht filtriert, das Lösungsmittel abgedampft und der Rückstand in Hexan verrieben. Anschliessend wird der Niederschlag filtriert und getrocknet. Man erhält so 4,7 g (76%) 3-Amino-5-chlor-2-fluor-pyridin, Smp. 74°C.

In analoger Weise erhält man die in den anschliessenden Tabellen aufgelisteten Zwischenprodukte der Formeln II und III des erfindungsgemässen Verfahrens.

Tabelle 1

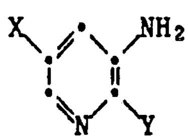

| X | Y |
|---|---|
| Cl | Br |
| Br | Cl |
| Br | Br |
| F | Cl |
| F | Br |
| $CF_3$ | F |
| $CF_3$ | Br |

Tabelle 2

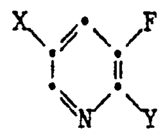

| X | Y |
|---|---|
| Cl | Br |
| Br | Cl |
| Br | Br |
| F | Cl |
| F | Br |
| $CF_3$ | F |
| $CF_3$ | Br |

**Patentansprüche**

1. Verfahren zur Herstellung von 2,3-Difluorpyridinen der Formel I

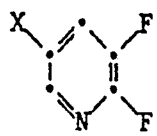 (I),

worin X für Halogen oder Trifluormethyl steht, dadurch gekennzeichnet, dass man ein 3-Amino-2-halogenpyridin der Formel II

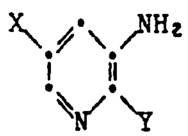 (II),

worin X die unter Formel I gegebene Bedeutung hat und Y für Brom, Chlor oder Fluor steht, durch Diazotieren in Gegenwart von Fluorwasserstoff in einem inerten Lösungsmittel in ein 3-Fluor-2-halogenpyridin der Formel III

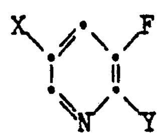 (III),

worin X und Y die unter Formel II gegebene Bedeutung haben, überführt, und erhaltene 3-Fluor-2-halogenpyridine der Formel III, in welchen Y Brom oder Chlor bedeutet, mit einem Fluorierungsmittel in Gegenwart einer katalytischen Menge eines Gemischs aus Cäsiumfluorid und einem Kronenäther behandelt.

2. Verfahren zur Herstellung von 2,3-Difluorpyridinen der Formel I

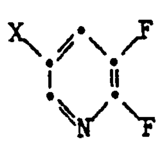 (I),

worin X für Halogen oder Trifluormethyl steht, dadurch gekennzeichnet, dass man ein 3-Amino-2-halogenpyridin der Formel II

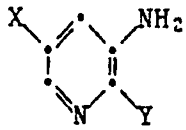

(II),

worin X die unter Formel I gegebene Bedeutung hat und Y für Brom oder Chlor steht, in Gegenwart von Fluorwasserstoff in einem inerten Lösungsmittel diazotiert und das entstandene 3-Fluor-2-halogenpyridin der Formel III

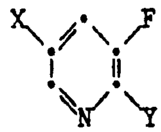

(III),

worin X und Y die unter Formel II gegebene Bedeutung haben, mit einem Fluorierungsmittel in Gegenwart einer katalytischen Menge eines Gemischs aus Cäsiumfluorid und einem Kronenäther behandelt.

3. Verfahren gemäss Anspruch 1 oder 2, dadurch gekennzeichnet, dass X für Chlor und Trifluormethyl steht.

4. Verfahren gemäss Anspruch 1, dadurch gekennzeichnet, dass Y für Fluor oder Chlor steht.

5. Verfahren gemäss Anspruch 2, dadurch gekennzeichnet, dass Y für Chlor steht.

6. Verfahren gemäss Anspruch 1, dadurch gekennzeichnet, dass X für Chlor oder Trifluormethyl und Y für Chlor oder Fluor stehen.

7. Verfahren gemäss Anspruch 2, dadurch gekennzeichnet, dass X für Chlor oder Trifluormethyl und Y für Chlor steht.

8. Verfahren gemäss Anspruch 1 oder 2, dadurch gekennzeichnet, dass man als Fluorierungsmittel Kaliumfluorid verwendet.

9. Verfahren gemäss Anspruch 1 oder 2, dadurch gekennzeichnet, dass man die Diazotierung mit einem Ueberschuss von Fluorwasserstoff ausführt.

10. Verfahren gemäss Anspruch 1 oder 2, dadurch gekennzeichnet, dass man die Diazotierung und Zersetzung bei einer Temperatur zwischen 0°C und +70°C unter Normaldruck durchführt.

11. Verfahren gemäss Anspruch 1 oder 2, dadurch gekennzeichnet, dass man als Diazotierungsmittel Natriumnitrit oder Distickstofftrioxid verwendet.

12. Verfahren gemäss Anspruch 1 oder 2, dadurch gekennzeichnet, dass man die Diazotierung mittels Natriumnitrit oder Distickstofftrioxid in Fluorwasserstoff mit einem inerten Lösungsmittel bei einer Temperatur zwischen 0°C und +70°C unter Normaldruck ausführt.

13. Verfahren gemäss Anspruch 1 oder 2, dadurch gekennzeichnet, dass man die Substitution von Chlor oder Brom in 2-Stellung des Pyridinringes durch Fluor in einem polaren aprotischen Lösungsmittel durchführt.

14. Verfahren gemäss Anspruch 13, dadurch gekennzeichnet, dass man als Lösungsmittel Dimethylsulfoxid, Dimethylsulfon, Sulfolan, Dimethylacetamid, N-Methylpyrrolidinon oder Dimethylformamid verwendet.

15. Verfahren gemäss Anspruch 1 oder 2, dadurch gekennzeichnet, dass man die Substitution von Chlor oder Brom in 2-Stellung des Pyridinringes durch Fluor bei einer Temperatur zwischen 80°C und 220°C durchführt.

16. Verfahren gemäss Anspruch 15, dadurch gekennzeichnet, dass die Temperatur zwischen 120°C und 170°C liegt.

17. Verfahren gemäss Anspruch 1 oder 2, dadurch gekennzeichnet, dass man die Substitution von Chlor oder Brom in 2-Stellung des Pyridinringes durch Fluor umittelbar nach der Diazotierung des 3-Amino-2-halogenpyridins der Formel II ohne Isolierung des 3-Fluor-2-halogenpyridins der Formel III bei einer Temperatur zwischen 80°C und 170°C durchführt.

18. Verfahren gemäss Anspruch 1 oder 2, dadurch gekennzeichnet, dass man dem Fluorierungsmittel Kaliumfluorid, 0,001 Mol bis 0,1 Mol an Fluorierungskatalysator pro Mol zu fluorierendes Ausgangsmaterial zusetzt.

19. Verbindungen der Formel IIa

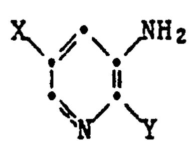

(IIa),

worin X für Halogen und Y für Brom, Chlor oder Fluor stehen, mit Ausnahme von 3-Amino-2,5-dichlorpyridin, 3-Amino-2,5-dibrompyridin, 3-Amino-2-chlor-5-brompyridin und 3-Amino-2-brom-5-chlorpyridin.

20. Verbindungen der Formel IIIa

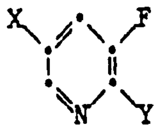

(IIIa),

worin X für Halogen und Y für Brom oder Chlor stehen, mit Ausnahme von 2,3,5-Trifluorpyridin, 2,3-Difluor-5-chlorpyridin und 2,3-Difluor-5-brompyridin.

21. 2,5-Dichlor-3-fluor-pyridin gemäss Anspruch 20.

22. 3-Amino-5-chlor-2-fluorpyridin gemäss Anspruch 19.

**Claims**

1. A process for the preparation of 2,3-difluoropyridines of formula I

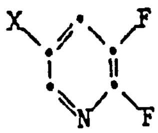

(I),

wherein X is halogen or trifluoromethyl, which process comprises converting a 3-amino-2-halopyridine of formula II

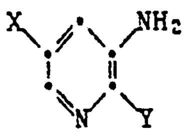

(II),

wherein X is as defined for formula I and Y is bromine, chlorine or fluorine, by diazotisation in an inert solvent, in the presence of hydrogen fluoride, into a 3-fluoro-2-halopyridine of formula III

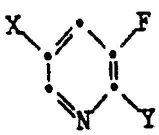

(III),

wherein X and Y are as defined for formula II, and treating resultant 3-fluoro-2-halopyridines of formula III, wherein Y is bromine or chlorine, with a fluorinating agent in the presence of a catalytic amount of a mixture of caesium fluoride and a crown ether.

2. A process for the preparation of 2,3-difluoropyridines of formula I

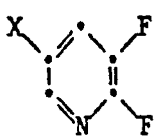

(I),

wherein X is halogen or trifluoromethyl, which process comprises diazotising a 3-amino-2-halopyridine of formula II

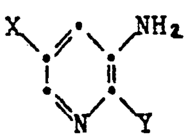

(II),

wherein X is as defined for formula I and Y is bromine or chlorine, in an inert solvent, in the presence of hydrogen fluoride, and treating the resultant 3-fluoro-2-halopyridine of formula III

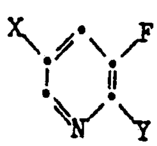

(III),

wherein X and Y are as defined for formula II, with a fluorinating agent in the presence of a catalytic amount of a mixture of caesium fluoride and a crown ether.

3. A process according to claim 1 or 2, wherein X is chlorine or trifluoromethyl.

4. A process according to claim 1, wherein Y is fluorine or chlorine.

5. A process according to claim 2, wherein Y is chlorine.

6. A process according to claim 1, wherein X is chlorine or trifluoromethyl and Y is chlorine or fluorine.

7. A process according to claim 2, wherein X is chlorine or trifluoromethyl and Y is chlorine.

8. A process according to claim 1 or 2, which comprises the use of potassium fluoride as fluorinating agent.

9. A process according to claim 1 or 2, which comprises carrying out the diazotisation with an excess of hydrogen fluoride.

10. A process according to claim 1 or 2, which comprises carrying out the diazotization and decomposition at a temperature in the range from 0°C to +70°C and under normal pressure.

11. A process according to claim 1 or 2, which comprises the use of sodium nitrite of dinitrogen trioxide as diazotising agent.

12. A process according to claim 1 or 2, which comprises carrying out the diazotisation using sodium nitrite or dinitrogen trioxide in hydrogen fluoride with an inert solvent at a temperature in the range from 0°C to +70°C and under normal pressure.

13. A process according to claim 1 or 2, which comprises carrying out the substitution of chlorine or bromine in the 2-position of the pyridine ring by fluorine in a polar aprotic solvent.

14. A process according to claim 13, which comprises the use of dimethyl sulfoxide, dimethyl sulfone, sulfolane, dimethylacetamide, N-methylpyrrolidinone or dimethylformamide as solvent.

15. A process according to claim 1 or 2, which comprises carrying out the substitution of chlorine or bromine in the 2-position of the pyridine ring by fluorine at a temperature in the range of from 80°C to 220°C.

16. A process according to claim 15, wherein the temperature is in the range from 120°C to 170°C.

17. A process according to claim 1 or 2, which comprises carrying out the substitution of chlorine or bromine in the 2-position of the pyridine ring by fluorine directly after the diazotization of the 3-amino-2-halopyridine of formula II, without isolation of the 3-fluoro-2-halopyridine of formula III and at a temperature in the range from 80°C to 170°C.

18. A process according to claim 1 or 2, which comprises adding to the fluorinating agent potassium fluoride 0.001 mol to 0.1 mol of fluorination catalyst per mol of starting material to be fluorinated.

19. Compounds of formula IIa

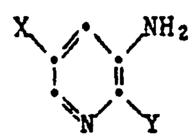

(IIa),

wherein X is halogen and Y is bromine, chlorine or fluorine, with the exception of 3-amino-2,5-dichloropyridine, 3-amino-2,5-dibromopyridine, 3-amino-2-chloro-5-bromopyridine and 3-amino-2-bromo-5-chloropyridine.

20. Compounds of formula IIIa

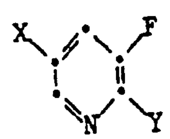

(IIIa),

wherein X is halogen and Y is bromine or chlorine, with the exception of 2,3,5-trifluoropyridine, 2,3-difluoro-5-chloropyridine and 2,3-difluoro-5-bromopyridine.

21. 2,5-Dichloro-3-fluoropyridine according to claim 20.

22. 3-Amino-5-chloro-2-fluoropyridine according to claim 19.

## Revendications

1. Procédé pour la préparation des 2,3-difluoropyridines de formule I

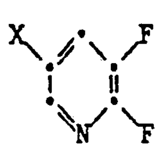

(I),

où X représente un halogène ou le trifluorométhyle, caractérisé en ce que l'on transforme une 3-amino-2-halogénopyridine de formule II

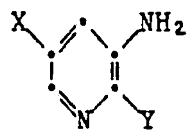

(II),

où X a la signification donnée à propos de la formule I et Y représente le brome, le chlore ou le fluor, par diazotation en présence d'acide fluorhydrique dans un solvant inerte, en une 3-fluoro-2-halogénopyridine de formule III

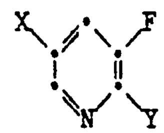

(III),

où X et Y ont la signification donnée à propos de la formule II et en ce que l'on traite les 3-fluoro-2-halogénopyridines de formule III dans laquelle Y représente le brome ou le chlore, avec un agent de fluoration, en présence d'une quantité catalytique d'un mélange de fluorure de césium et d'un éther couronne.

2. Procédé pour la préparation des 2,3-difluoropyridines de formule I

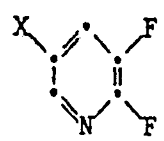

(I),

où X représente un halogène ou le trifluorométhyle, caractérisé en ce que l'on diazote une 3-amino-2-halogénopyridine de formule II

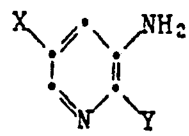

(II),

où X a la signification donnée à propos de la formule I et Y représente le brome ou le chlore, en présence d'acide fluorhydrique, dans un solvant inerte et en ce que l'on traite la 3-fluoro-2-halogénopyridine obtenue de formule III

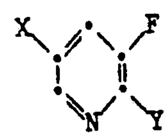

(III),

où X et Y ont la signification donnée à propos de la formule II, avec un agent de fluoration, en présence d'une quantité catalytique d'un mélange de fluorure de césium et d'un éther couronne.

3. Procédé selon la revendication 1 ou 2, caractérisé en ce que X représente le chlore ou le trifluorométhyle.

4. Procédé selon la revendication 1, caractérisé en ce que Y représente le fluor ou le chlore.

5. Procédé selon la revendication 2, caractérisé en ce que Y représente le chlore.

6. Procédé selon la revendication 1, caractérisé en ce que X représente le chlore ou le trifluorométhyle et Y représente le chlore ou le fluor.

7. Procédé selon la revendication 1, caractérisé en ce que X représente le chlore ou le trifluorométhyle et Y représente le chlore.

8. Procédé selon la revendication 1 ou 2, caractérisé en ce que l'on utilise comme agent de fluoration le fluoration le fluorure de potassium.

9. Procédé selon la revendication 1 ou 2, caractérisé en ce que l'on effectue la diazotation avec un excès d'acide fluorhydrique.

10. Procédé selon la revendication 1 ou 2, caractérisé en ce que l'on effectue la diazotation et la décomposition à une température entre 0°C et +70°C sous une pression normale.

11. Procédé selon la revendication 1 ou 2, caractérisé en ce que l'on utilise comme agent de diazotation le nitrite de sodium ou le trioxyde d'azote.

12. Procédé selon la revendication 1 ou 2, caractérisé en ce que l'on effectue la diazotation par le nitrite de sodium ou par le trioxyde d'azote dans l'acide fluorhydrique avec un solvant inerte à une température ente 0°C et +70°C sous une pression normale.

13. Procédé selon la revendication 1 ou 2, caractérisé en ce que l'on effectue la substitution du chlore ou du brome en position 2 du noyau pyridine par le fluor dans un solvant polaire aprotique.

14. Procédé selon la revendication 13, caractérisé en ce que l'on utilise comme solvant le diméthylsulfoxyde, le diméthylsulfone, le sulfolane, la diméthylacétamide, le N-méthylpyrrolidinone ou le diméthylformamide.

15. Procédé selon la revendication 1 ou 2, caractérisé en ce que l'on effectue la substitution du chlore ou du brome en position 2 du noyau pyridine par le fluor à une température entre 80°C et 220°C.

16. Procédé selon la revendication 15, caractérisé en ce que la température est comprise entre 120°C et 170°C.

17. Procédé selon la revendication 1 ou 2, caractérisé en ce que l'on effectue la substitution du chlore ou du brome en position 2 du noyau pyridine par du fluor, immédiatement après la diazotization de la 3-amino-2-halogénopyridine de formule II sans isolation du 3-fluoro-2-halogénopyridine de formule III à une température entre 80°C et 170°C.

18. Procédé selon la revendication 1 ou 2, caractérisé en ce que l'on additionne à l'agent de fluoration le fluorure de potassium, 0,001 mole à 0,1 mole d'un catalyseur de fluoration par mole de matériau de départ à fluorer.

19. Composés de formule IIa

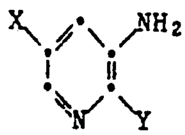

(IIa),

où X représente un halogène et Y représente le brome, le chlore ou le fluor, à l'exception de la 3-amino-2,5-dichloropyridine, de la 3-amino-2,5-dibromopyridine, de la 3-amino-2-chloro-5-bromopyridine et de la 3-amino-2-bromo-5-chloropyridine.

20. Composés de formule IIIa

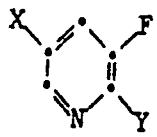

(IIIa),

où X représente un halogène et Y représente le brome ou le chlore, à l'exception de la 2,3,5-trifluoropyridine, de la 2,3-difluoro-5-chloropyridine et de la 2,3-difluoro-5-bromopyridine.

21. La 2,5-dichloro-3-fluoropyridine selon la revendication 20.

22. La 3-amino-5-chloro-2-fluoropyridine selon la revendication 19.